# EUROPÄISCHE PATENTSCHRIFT

(11) **EP 0 504 798 B1**
(45) Veröffentlichungstag und Bekanntmachung des Hinweises auf die Patenterteilung: **13.11.1996**
(21) Anmeldenummer: 92104583.7
(22) Anmeldetag: 17.03.1992
(51) Int. Cl.: C12N 9/80, C12N 15/55, C12P 21/00

(54) **Verfahren zur Herstellung von Glutarylacylase in grossen Mengen**
Method of production of high amounts of glutaryl acyclase
Méthode de production de la glutaryl acyclase en grande quantité

(30) Priorität: 18.03.1991 DE 4108823; 05.11.1991 DE 4136389
(43) Veröffentlichungstag der Anmeldung: 23.09.1992
(73) Patentinhaber: HOECHST AKTIENGESELLSCHAFT, 65926 Frankfurt am Main (DE)
(72) Erfinder: Koller, Klaus-Peter, Dr., W-6232 Bad Soden am Taunus (DE); Riess, Günther Johannes, Dr., W-6234 Hatteresheim am Main (DE); Aretz, Werner, Dr., W-6240 Königstein/Taunus (DE)

(56) Entgegenhaltungen:
- EP-A- 0 393 684
- FR-A- 2 552 102
- JOURNAL OF BACTERIOLOGY, Bd. 163, Nr. 3, 1985, BALTIMORE US, Seiten 1222 - 1228; A.MATSUDA ET AL.: 'Molecular cloning and structure of the gene for 7 beta -4- carboxybutanamido cephalosporanic acid acylase from a Pseudomonas strain'
- AGRICULTURAL AND BIOLOGICAL CHEMISTRY, Bd. 45, Nr. 7, 1981, TOKYO JP, Seiten 1561 - 1567; YUZO SHIBUYA ET AL.: 'Isolation and properties of 7 beta - 4 - carboxybutanamido cephalosporanic acid acylase-producing bacteria'
- P.H.POUWELS ET AL. 'Cloning vectors' 1988 , ELSEVIERS , AMSTERDAM

## Beschreibung

Bei der enzymatischen Herstellung von 7-Aminocephalosporansäure aus Cephalosporin C wird zunächst 7β-(4-Carboxybutanamido)-Cephalosporansäure (Glutarylamidocephalosporansäure) gebildet, die mit Hilfe des Enzyms Glutarylacylase (bzw. Glutarylamidase), im folgenden als "GA" bezeichnet, zu 7-Aminocephalosporansäure deacyliert wird.

Die gentechnische Herstellung von GA in E. coli wurde bereits von R. Matsuda und K.-l. Komatsu, J. Bact. 163 (1985) 1222-1228 beschrieben. Die spezifische Aktivität der GA konnte jedoch nur geringfügig gesteigert werden (von 0,12 Einheiten des eingesetzten Stammes auf 0,2 Einheiten pro mg).

Matsuda et al., a.a.O., verwendeten für ihre Untersuchungen eine Mutante Pseudomonas Sp. GK 16, für die keine Hinterlegungsbezeichnung angegeben ist. Die Autoren gehen aus von einem Stamm Pseudomonas sp. SY-77-1, der beim Fermentation Research Institute (FRI) unter der Bezeichnung FERM 2410 erhältlich ist.

Es wurde nun gefunden, daß aus einem Pseudomonas-Stamm das GA codierende Gen isoliert werden kann und daß mit Hilfe dieses Gens das Enzym in E. coli mit einer mindestens fünffach höheren spezifischen Aktivität gewonnen werden kann. Dieses aus dem Pseudomas-Stamm isolierte Enzym ist mit der GA aus dem hinterlegten Stamm vergleichbar.

Bevorzugte Ausgestaltungen sowie weitere Aspekte der Erfindung werden im folgenden näher erläutert und in den Patentansprüchen definiert.

Es zeigte sich, daß die Klonierung des Gens in einen "high copy number vector" wie pUC18 unter den üblichen Induktionsbedingungen zu einer Abtötung der transformierten Zellen führt. Es wurden deshalb für folgenden Untersuchungen "low copy number vectors" konstruiert, mit denen eine Genbank des Genoms des Stammes angelegt wurde. Diese Vectoren, plac10, plac100 und plac200, enthalten den Replikationsursprung des bekannten Plasmids pACYC 184 (A.C.Y. Chang u.S.N. Cohen, J. Bact. 134 (1978) 1141-1156) und liegen deshalb in etwa fünf Kopien je Zelle vor. Diese Vectoren erlauben eine Selektion mit Chloramphenicol und so die Vermeidung einer β-Lactamase-Bildung, die auf Cephalosporin einwirken könnte.

Zur Identifizierung des GA-Gens wurde zunächst eine Genbank angelegt, die das total mit Pstl verdaute Genom enthielt. Es wurde mit Hilfe von 2 Sonden, die den Kodons 30 bis 40 und 41 bis 51 der Veröffentlichung von Matsuda et al., a.a.O., entsprechen, ein Klon gefunden, der ein 3,7 kb Pstl-lnsert enthielt. Dieses wurde nach Umklonieren in den Phagen M13mp19 ansequenziert. Dabei ergab sich eine mehr als 98 %ige Übereinstimmung mit der publizierten Sequenz im Bereich der Aminosäuren 51 bis 209. Das 3,7 kb Pstl-Fragment wurde nun radioaktiv markiert als Sonde benutzt, um eine Plasmidgenbank zu "screenen", die durch Klonierung von partiell Pstl-geschnittener DNA des Stammes in den "low copy number vector" plac 100 konstruiert worden war. Durch Restriktionsanalyse und Vergleich der Schnittstellen mit den publizierten Daten ergab es sich, daß der Klon pCM 145 das komplette Gen für GA sowie 5'- und 3'-flankierende Sequenzen enthält. Dieser Klon enthält den Vector plac 100 mit einem ca. 10 kb Pstl-lnsert. Er produziert etwa 4 U/l GA. Dieser Klon wurde am 08.03.1991 unter den Bedingungen des Budapester Vertrags bei der Deutschen Sammlung von Mikroorganismen und Zellkulturen GmbH, Braunschweig, unter der Hinterlegungs-Nr. DSM 6409 hinterlegt.

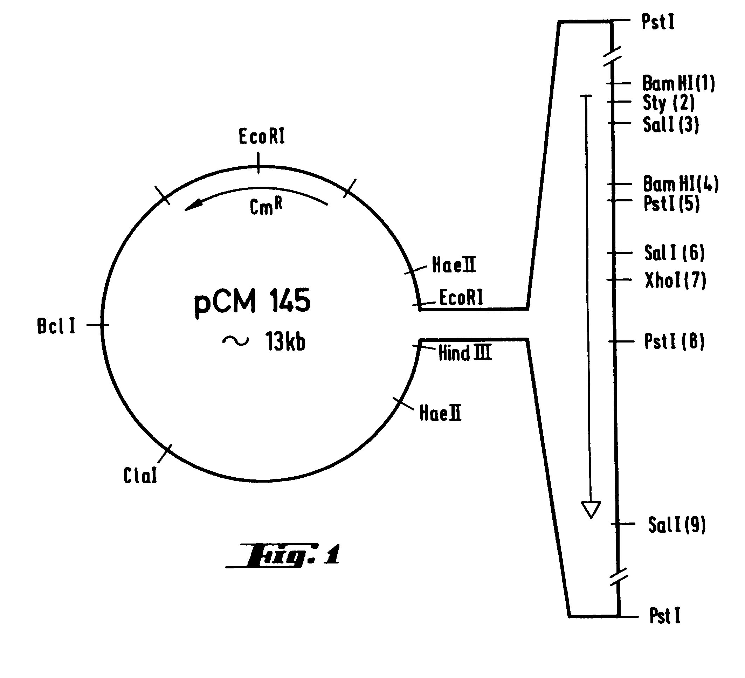

Die Figur 1 zeigt eine Restriktionskarte des Plasmids pCM 145. Im Insert sind die für die Klonierungen wichtigen Restriktionsschnittstellen numeriert.

Durch Umklonieren und Subklonierung konnten die GA-Ausbeuten noch bis zu etwa 450 U/l gesteigert werden.

Überraschenderweise zeigte es sich dabei, daß die Reduktion der Fermentationstemperatur von 37 °C bis zu 23 °C, vorzugsweise 25 - 30 °C, insbesondere 27 - 28 °C, eine deutliche Verbesserung der Ausbeute erlaubt. Bei dieser Temperatur waren Fermentationen über längere Zeit möglich, während die Induktion des E. coli-Systems mit IPTG bei 37 °C für den Wirtsstamm lethal war.

Die Induktion erfolgt vorzugsweise in der logarithmischen Wachstumsphase, insbesondere in der späten logarithmischen Phase. Als Wirtsstämme werden vorzugsweise esterasefreie oder -arme E. coli K 12-Stämme eingesetzt, da so die Aufarbeitung erheblich erleichtert wird.

Die Produktivität kann vom Wirtsstamm beeinflußt werden. Als geeignet erwiesen sich beispielsweise die Stämme E. coli MC 1061 (ATCC 53338), E. coli W 3110 (ATCC 27325) oder E. coli DH1 (ATCC 33849).

Für die Fermentation können die bekannten Mineralmedien und komplexen Medien mit Hefeextrakt, Pepton, Trypton oder "Casamino Acids" eingesetzt werden. Die günstigsten Bedingungen kann der Fachmann durch einfache Vorversuche leicht ermitteln.

Der durch Umklonierung nachfolgend (Beispiel 4) beschriebene Expressionsvektor T 307 ergab bereits bei Verwendung des E. coli Stammes TG1 (Firma Amersham-Buchler, Braunschweig) unter Induktionsbedingungen eine Ausbeute von ca. 1000 U/l Kulturmedium. Das Plasmid T 307 weist ein β-Lactamase-Gen auf, dessen Genprodukt, das Enzym β-Lactamase, zur Selektion rekombinanter Klone dient. Bei der β-Lactamase handelt es sich wie bei der GA um ein sekretiertes, periplasmatisch lokalisiertes Enzym. Überraschenderweise konnte nun gezeigt werden, daß der Ersatz von Teilen des β-Lactamase-Strukturgens durch ein Chloramphenicol-Resistenzgen zu deutlich verbesserten Ausbeuten führt (vergl. Beispiel 9, Plasmid T 347). Die Resistenz gegen Chloramphenicol wird durch das Enzym Acetyltransferase (CAT) vermittelt, das im Gegensatz zur β-Lactamase intrazellulär lokalisiert ist.

Eine weitere Verbesserung der Ausbeute konnte erzielt werden, wenn man nicht nur Teile des β-Lactamase-Strukturgens entfernt, sondern den gesamten Strukturgenbereich, vor allem den kodierenden Bereich des für die Sekretion der β-Lactamase verantwortlichen Signalpeptids mit entfernt und als Selektionsmarker ebenfalls das Chloramphenicol-Acetyltransferase-Gen einsetzt (vergl. Beispiel 10, Plasmid T 363).

Die Erfindung betrifft folglich auch den Einsatz von Expressionsvektoren, die als Selektionsgen kein Gen für ein sekretiertes, periplasmatisch lokalisiertes Genprodukt bzw. Enzym enthalten. Dann können auch "high copy" Vektoren eingesetzt werden, wobei vorzugsweise Vektoren ohne ein β-Lactamasegen gewählt werden. Es wurde weiter gefunden, daß optimale Ausbeuten bei Verwendung der E. coli K 12 Stämme W 3110 M bzw. MC 1061 erreicht werden. Hierbei werden diese Plasmide auch nicht im Lauf der Fermentation eliminiert sondern sind stabil.

Es ist aber auch möglich, anstelle eines intrazellulär aktiven Selektionsmarkers einen periplasmatisch oder membranständig lokalisierten Marker zu verwenden, wenn es gelingt, die Expression des Markers während der Produktionsphase für die Glutarylamidase soweit zu vermindern, daß sie nicht zur Instabilität des Plasmids führt. Geeignet ist zum Beispiel das Tetracyclin-Resistenzgen von Tu 1721, das sehr streng reguliert ist (Klock, G. et al., J. Bacteriol. 161, 326 - 332, 1988) oder vergleichbar streng regulierte für periplasmatisch oder membranständig lokalisierte Marker kodierende Gene.

Im Folgenden werden die zur Erreichung der hohen Ausbeuten notwendigen Konstruktionen der Expressionsvektoren näher beschrieben. Die verwendeten Enzyme wurden von New England Biolabs (Schwalbach) bzw. von Gibco/BRL (Eggenstein) bezogen und gemäß den Vorschriften der Hersteller verwendet. Nicht im einzelnen beschriebene gentechnische Arbeiten wurden gemäß den detaillierten Anleitungen bei Ausubel et al., Current Protocols in Molecular Biology, Greene Publishing Associates and Wiley Interscience, 1987 und Supplements durchgeführt. Alle Angaben hinsichtlich der Größe der Plasmide (bp) sind ca. Angaben.

Einzelheiten der Erfindung werden in den folgenden Beispielen erläutert. Prozentangaben beziehen sich auf das Gewicht, sofern keine anderen Angaben gemacht sind.

### Beispiele:

### 1. Herstellung des Plasmids plac10

Aus dem handelsüblichen Plasmid pBR 325 (SIGMA) wird das 1,1 kb EcoRI-HindIII-Fragment isoliert und mit dem 1,5 kb EcoRI-HindIII-Fragment aus dem Vektor pACYC 184 ligiert. Das so erhaltene "Mini-pACYC" wird partiell mit Haell verdaut und mit dem 819 bp Haell-Fragment aus dem handelsüblichen Vector pUC12, das den Polylinker und die α-Komplementation von lac Z enthält, ligiert. Vectoren, die das Insert in die Haell-Stelle aus dem pACYC 184-Fragment in der Anordnung enthielten, daß der Polylinker die Schnittstellen in der Reihenfolge EcoRI-HindIII, gefolgt vom Replikationsursprung, enthielt, erhielten die Bezeichnung plac10.

### Beispiel 2: Herstellung der Vektoren plac100 und plac200

Das im Beispiel 1 beschriebene "Mini-pACYC" enthält an der Hindlll-Schnittstelle 2 benachbarte Schnittstellen für Clal. Durch Entfernung dieses Clal-Fragments erhält man aus plac10 den Vektor plac100, der die für die Ligierung zu "Mini-pACYC" verwendete Hindlll-Schnittstelle nicht mehr enthält.

Verdaut man analog Beispiel 1 plac100 partiell mit Haell und ersetzt das Haell-Fragment mit dem Polylinker aus pUC 12 durch das entsprechende Fragment aus pUC 18, so erhält man den Vector plac200.

### 3. Anlegen einer Genbank

Die gesamte DNA des Pseudomonas-Stammes wurde mit Pstl total verdaut und in das mit Pstl geschnittene plac 10 ligiert. Mit dem Ligierungsgemisch wurde E. coli TG1 (Pharmacia) transformiert.

Zum Screening wurden zunächst 2 Oligonukleotide eingesetzt, die den Kodons 30 bis 40 und 41 bis 50 der publizierten GA-Sequenz entsprechen. Hierdurch wurde der Klon pWK 96 isoliert, der ein 3,7 kb PstI-Insert enthält. Dieses hybridisierte mit beiden Sonden. Durch Umklonieren in den Phagen M13mp19 und Ansequenzieren ergab sich eine über 98 %ige Übereinstimmung mit der publizierten DNA-Sequenz im Bereich der Aminosäuren 150 bis 209. Der Vergleich mit der publizierten Genkarte zeigt eine Übereinstimmung in den relevanten Schnittstellen.

Das Insert wurde radioaktiv markiert und seinerseits als Sonde für eine Genbank, die aus partiell mit Pstl verdauter Gesamt-DNA angelegt wurde, verwendet, wobei der Klon pCM 145 gefunden wurde, der ein ca. 10 kb Insert enthält. Die Restriktionsanalyse und ein Vergleich mit den publizierten Schnittstellen ergibt, daß das komplette Gen sowie die 5'- und 3'-flankierenden Sequenzen kloniert wurden. Der Klon liefert eine Ausbeute von 4 U/l GA. Er dient als Ausgangsmaterial für die folgenden Arbeiten.

### 4. Umklonierungen

Das Plamid plac 100 wird mit EcoRI und Hindlll geschnitten und das Insert mit dem GA-Gen in den mit den gleichen Enzymen geöffneten handelsüblichen Vektor pUC 19 umkloniert. Der so erhaltene Klon T 286 liefert eine Ausbeute von 23,5 U/l GA. Hierbei wird ein Teil des Enzyms sekretiert (im Ausgangsstamm wird das Enzym nur im Periplasma gefunden).

Zur Entfernung von Sequenzen, die die Expression des Gens in E. coli möglicherweise behindern, wurde - in Anlehnung an die publizierte Karte des GA-Gens - das Gen rekonstruiert. Hierbei wird auf die Nummern der Restriktions-Schnittstellen in Figur 1 Bezug genommen.

Zunächst wurde das 0,8 kb große BamHI-Fragment (BamHI(1)-BamHI(4)), das eine Sall-Schnittstelle trägt, in das handelsübliche Plasmid pUC 18 kloniert. In das entstandene Plasmid, das mit Sall geschnitten wurde, wird ein ebenfalls 0,8 kb großes Sall-Fragment (SaII(3)-SaII(6)) eingefügt. In das neue Plasmid, das mit Pstl geschnitten wurde, wird ein 0,9 kb großes Pstl-Fragment (Pstl(5)-Pstl(8)) kloniert, wobei der Klon pT 93 erhalten wird. Weiterhin wurde ein 1,7 kb Sall-Fragment (Sall(6)-Sall(9)) in pUC 18 kloniert und auf richtige Orientierung überprüft; es entsteht das Plasmid pT 98. Da das klonierte Pstl-Fragment (Pstl(5)-Pstl(8)) eine Xhol-Schnittstelle enthält, konnte das komplette Gen zusammenkloniert werden, indem das EcoRI-Xhol-Fragment aus pT 93 mit dem größeren, auch den Replikationsursprung und das Resistenzgen enthaltenden Xhol-EcoRI-Fragment aus pT 98 verbunden wurde. Hierbei entsteht das Plasmid pT 103. Damit transformierte E. coli-Kulturen produzieren bis zu 130 U/l GA, wobei die Expression offenbar über eine 5'-seitige Promotoraktivität aus der Pseudomonas-DNA gesteuert wird.

Isoliert man aus dem Plasmid pT103 nach Verdauung mit den Restriktionsenzymen Sstl und Hindlll das Fragment mit dem GA-Gen und kloniert es in den mit den gleichen Enzymen geschnittenen Vector plac200, so resultiert der Expressionsvector pT104, mit dem eine GA-Ausbeute von über 1000 U/l erreicht wird.

Nach Umklonieren des rekonstruierten Gens in die EcoRI-HindIII-Schnittstellen des handelsüblichen Expressionsvektor pBtac (Hersteller: Boehringer-Mannheim) wird das Plasmid pT 105 erhalten. Damit transformierte E. coli-TG 1-Stämme produzieren nach 3-tägiger Fermentation bis 288 U/l GA im Schüttelkolben bei einer Fermentationstemperatur von 37° C.

Zur Einklonierung des GA-Gens in den Vektor pTrc 99 A (E. Amann et al., Gene 69 (1988) 301-315) wird ein synthetischer Linker (SEQ ID NO: 1) benötigt:

(NcoI)                                                    (StyI)
C  ATG CTG AGA GTT CTG CAC CGG GCG GCG TCC GC
        GAC TCT CAA GAC GTG GCC CGC CGC AGG CGG AAC

Aus dem Plasmid pCM 145 wird nach Verdauung mit den Enzymen Styl und BamHI ein 0,57 kb großes BamHI(4)-Styl(2)-Fragment isoliert (in der publizierten DNA-Sequenz findet sich die Styl-Schnittstelle im Bereich der Aminosäuren 11 bis 13). Dieses Fragment und der vorstehend genannte Linker werden in das mit Ncol und BamHI geschnittene Plasmid pTrc 99 A ligiert, wobei das Plasmid T 297 unter Verlust der Ncol-Schnittstelle erhalten wird.

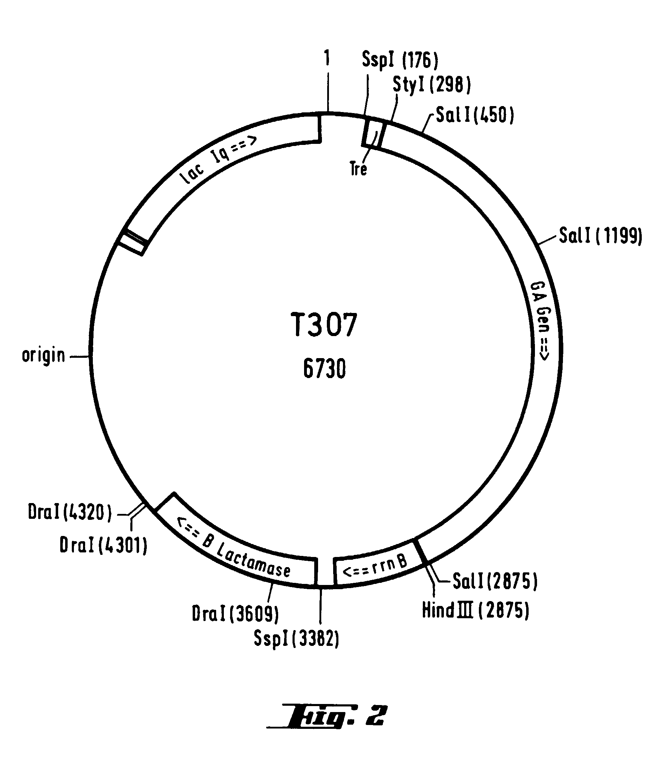

Weiterhin werden aus dem Plasmid pCM 145 ein 1,5 kb Sall-Fragment (Sall(6)-Sall(9)) sowie ein 0,34 kb BamHI-Sall-Fragment isoliert und in den Vektor pUC 18 ligiert, der mit Sall und BamHI geöffnet wurde. Hierbei resultiert das Plasmid T 306, das auf richtige Orientierung des Sall(6-9)-Fragments überprüft wurde. Aus dem Plasmid T 306 wird mit den Enzymen BamHI und Hindlll ein 2,1 kb großes Fragment isoliert (das das Fragment ab der BamHI (4)- bis zur Sall(9)-Stelle umfaßt). Dieses Fragment wurde in den mit den Enzymen BamHI und Hindlll geöffneten Vektor T 297 hineinligiert, wobei das Plasmid T 307 (Figur 2) erhalten wird. Die damit transformierte E. coli Population produziert nach 2-tägiger Fermentation bei 28 °C bis 260 U/l GA. Unter geänderten Fermentationsbedingungen (siehe folgende Beispiele) kann diese Ausbeute auf über 780 U/l gesteigert werden.

Eine Induktion des Systems mit IPTG bei 37 ° C ist für den transformierten E. coli-Stamm lethal, was offenbar auf den Einsatz des "high-copy-number-vector" T 307 zurückzuführen ist. Sie wird daher bei Temperaturen unterhalb 30 ° C durchgeführt.

### 5. GA-Fermentation mit E. coli-Klonen

Die E. coli-Klone DH1 T105 und TG1 T307 lassen sich auf Mineral- und Komplexmedien anziehen und produzieren bereits in der logarithmischen Wachstumsphase GA. Der maximale GA-Titer wird in der stationären Phase erreicht. Dies zeigt, daß die GA-Produktion von der Wachstumsgeschwindigkeit abhängig ist. In beiden Klonen liegt das Enzym zu 50 - 60 % cytoplasmatisch, zu 40 - 50 % periplasmatisch und zu maximal 10 % im Kulturfiltrat vor.

Zur kompletten Freisetzung der GA müssen die Zellen mittels Ultraschall, "French Press" oder einer "Dyno Mill" aufgeschlossen werden. Die periplasmatische GA kann durch Detergentien wie Cetyltrimethylammoniumchlorid oder Toluol solubilisiert werden. Ein Zusatz von EDTA und Lysozym erhöht die Effizienz. Einleitende Untersuchungen zur Optimierung der Fermentationsparameter zeigen, daß die Klone im Temperaturbereich von 25 ° C bis 37 ° C wachsen und produzieren können. Bei Temperaturen von über 30 ° C muß der Sauerstoffpartialdruck jedoch bei <5 % gehalten werden. Eine Wachstumstemperatur von 28 ° C führt zu einem für die GA-Produktion erforderlichen Anstieg der Verdopplungszeit auf >2 Std.; unter diesen Bedingungen ist die GA-Bildung nicht mehr von einem geringen pO₂ abhängig. Eine deutliche Korrelation zwischen GA-Volumenproduktivität (U/l Kulturlösung) und Biomassekonzentration (g/l) wurde festgestellt.

### 6. GA-Produktion in Mineralmedium

Die Stammhaltung des Klones TG1 T307 erfolgt bei -18 ° C im YT-Glycerin-Medium:

| | |
|---|---|
| Glycerin | 17,0 % |
| Hefeextrakt | 0,7 % |
| Bactotrypton | 0,4 % |
| NaCl | 0,4 % |
| Ampicillin | 50µg/ml |

Aus dieser Suspension werden Agarplatten des gleichen Mediums angelegt, 24 Std. bei 28-37 ° C inkubiert und die Vorkultur mit einer Einzelkolonie inokuliert.

VK-Medium:

| | |
|---|---|
| Bactotrypton | 1 % |
| Hefeextrakt | 0,5 % |
| NaCl | 0,5 % |
| Ampicillin | 50µg/ml |
| pH = 7,2 | |

100 ml dieser Nährlösung in 300 ml Erlenmeyerkolben werden nach den Animpfen für 24 Std. bei 28 ° C und 220 Upm inkubiert. Die Kultur zeigt dann eine OD₅₇₈ₙₘ von 3,0.

Folgende Hauptkultur (25 ml Nährlösung/300 ml Kolben) wird mit 2 % der Vorkultur beimpft und 24 - 72 Std. bei 28 ° C und 220 Upm inkubiert:

| Mineralmedium: | |
|---|---|
| | Gew.-% |
| NaH₂ PO₄ | 1,110 |
| Na₂HPO₄ | 3,680 |
| KCl | 0,100 |
| (NH₄)₂SO₄ | 0,325 |
| MgSO₄.7H₂O | 0,200 |
| Citronensäure | 0,390 |
| Glycerin oder Glucose | 4,000 |
| Fe₂ (SO₄).₃H₂O | 0,0250 |
| Spurenelementlösung | 0,1000 |
| pH | 6,0-6,5 |

| Spurenelementlösung: | |
|---|---|
| | Gew.-% |
| H₃BO₃ | 0,208 |
| (NH₄)₆ Mo₇O₂₄ | 0,080 |
| ZnSO₄.7H₂O | 0,160 |
| MnSO₄.4H₂O | 0,160 |
| CuSO₄.5H₂O | 0,160 |
| KJ | 0,048 |

- Biomasse nach 50 Std.:: 11 g/l
- GA nach 50 Std.:: 39 U/l

### 7. GA-Produktion im Komplexmedium

Analog Beispiel 6 werden Stammhaltung und Vorkulturanzucht durchgeführt und ein 5 1-Fermenter mit folgendem Komplexmedium (31) angeimpft:

### Komplexmedium:

| | |
|---|---|
| Bactotrypton | 1 % |
| Hefeextrakt | 0,5 % |
| NaCl | 0,5 % |
| pH 7,2 | |

- Fermentationsparameter:: 28 ° C, 400 Upm; 0,5 vvm
- Fermentationsdauer:: 24 - 72 Std.
- Biomasse nach 72 Std.:: 7 g Feuchtmasse/l
- GA nach 72 Std.:: 460 U/l Kulturlösung; 66 U/g Feuchtmasse

### GA-Produktion im aufgestockten Komplexmedium

Analog Beispiel 7 wurde der Klon TG1 T307 in folgendem Medium angezogen: Komplexmedium:

| | |
|---|---|
| Bactotrypton | 2 % |
| Hefeextrakt | 1,0 % |
| NaCl | 0,5 % |
| pH 7,2 | |

- Fermentationsparameter:: 28 ° C, 500 Upm; 0,8 vvm
- Biomasse nach 53 Std.::
- 15 g Feuchtmasse/l
- GA nach 63 Std.:: 780 U/l Kulturlösung; 52 U/g Feuchtmasse

Dieses Beispiel zeigt den Zusammenhang zwischen der Biomasse- und Volumenproduktivitätssteigerung.

Wird analog Beispiel 7 der Stamm DH1 T104 angezüchtet, so erreicht man nach 70 Stunden eine Biomasse von 11 g Feuchtmasse/l und eine Volumenaktivität von 1040 U/l Kulturlösung mit 97 U/g Feuchtmasse. Dies entspricht einer spezifischen Aktivität von ca. 1 U/mg Protein.

### 8. Induzierbarkeit der GA-Produktion

Wird der Klon TG1 T307 in der späten logarithmischen Wachstumsphase mit IPTG (Endkonzentration 1 - 5 mM) induziert, so erhöhen sich die Ausbeuten um 60 % innerhalb von 5 - 10 Stunden.

### Beispiel 9: Plasmid T 347

Isoliertes Plasmid DNA aus dem Stamm TG1 (T 307) wird mit dem Enzym Dral komplett geschnitten und das größere Fragment, das den Replikationsursprung sowie das unter der Kontrolle des Trc-Promoters stehende GA-Gen enthält, mittels Elektroelution aus dem zur Trennung der Fragmente verwendeten 0,6 % Agarosegel isoliert.

Aus dem Vektor pACYC 184 (Chang und Cohen, J. Bacteriol. 134, 1141 - 1156, 1978) wird durch Verdauung mit dem Restrktionsenzym Hae II ein ca. 1,3 kb großes DNA-Fragment freigesetzt, welches das CAT-Gen sowie den regulatorischen Bereich für das Den enthält. Dieses Fragment wird nach Trennung im Agarose-Gel ebenfalls durch Elektroelution gewonnen. Durch das Enzym Hae II werden an den Enden des Fragments überhängende 3'-Enden geschaffen, die sich nicht für eine Ligation mit dem o. a. Dra I Vektorfragment eignen, das stumpfe Enden (blunt ends) enthält. Daher wurden die überhängenden Enden des Hae 11-Fragments mit der Exonuclease S1 abverdaut und das Fragment mit DNA-Polymerase 1 in Gegenwart der 4 Desoxynucleotid-Triphosphate nachbehandelt.

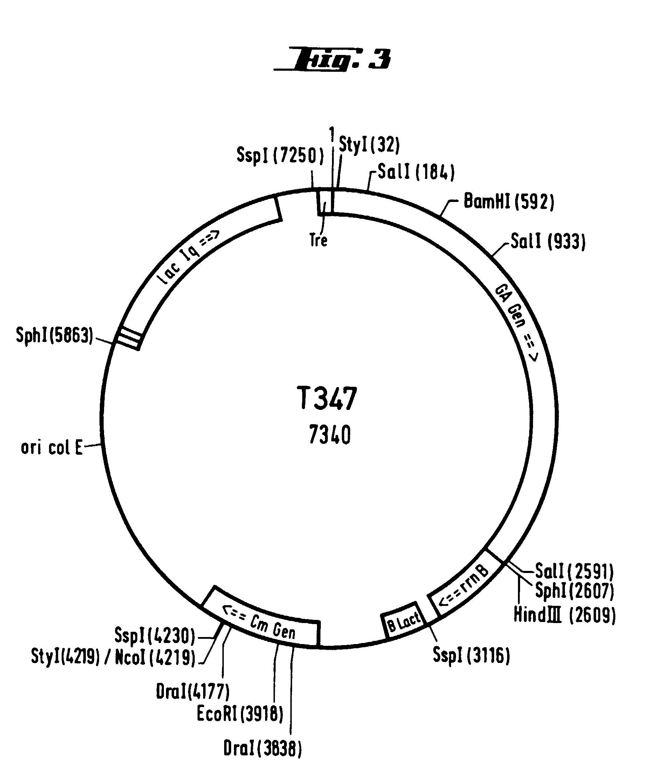

Das so vorbereitete Hae 11-Fragment wird mit Hilfe den Enzyms DNA-Ligase mit dem Dra 1-Vektorfragment zusammenligiert und in E. coli MC 1061 transformiert. Die Selektion geeigneter rekombinanter E. coli Klone erfolgt auf Chloramphenicol-Resistenz und Synthese des Enzyms GA hin. Die GA-Bildung wurde mit Hilfe des Schibuja-Testes ermittelt. Rekominante Klone enthalten das Plasmid T 347, dessen Restriktionskarte in Figur 3 wiedergegeben ist. Für die Höhe des Expression der GA ist die Orientierung des CAT-Gens im Vektor nicht entscheidend.

### Beispiel 10: Plasmid T 363

Bei längerer Fermentationsdauer von E. coli MC 1061 (T 347) hatte es sich gezeigt, daß die Zahl der Plasmid-tragenden Zellen nach Induktion der GA-Expression durch IPTG deutlich abnimmt. Als Grund für die Instabilität wird vermutet, daß über den noch vorhandenen β-Lactamase-Promoter ein ca. 69 Aminosäuren umfassendes, verkürztes β-Lactamase-Rumpfprotein gebildet wird, das das Signalpeptid und die Reste der β-Lactamase enthält. Da dessen Expression durch die Einklonierung des CAT-Gen nicht verhindert werden kann, führt dies wahrscheinlich zu einem negativen Selektionsdruck, d. h. Plasmidverlust. Um zu einem über die gesamte Fermentationsdauer stabilen Vektor zu gelangen, wurde das Plasmid T 307 mit den Enzymen Ssp I und Dra I doppelt verdaut. Die beiden größten Fragmente, die einerseits den Replikationsursprung, andererseits das GA-Den mit seinem Trc-Promoteranteil enthalten, wurden nach Auftrennung im 0,6 % Agarosegel durch Elektroelution isoliert. Gemäß Beispiel 9 wurde das Hae II-Fragment mit dem CAT-Gen hergestellt und mit den beiden o. a. Fragmenten mit Hilfe den Enzyms DNA-Ligase zusammengefügt. Das Ligationsgemisch wurde in den E. coli Stamm MC 1061 transformiert. Rekombinante Klone wurden selektioniert aufgrund ihrer Fähigkeit, in Gegenwart von Chloramphenicol zu wachsen und das Enzym GA zu bilden.

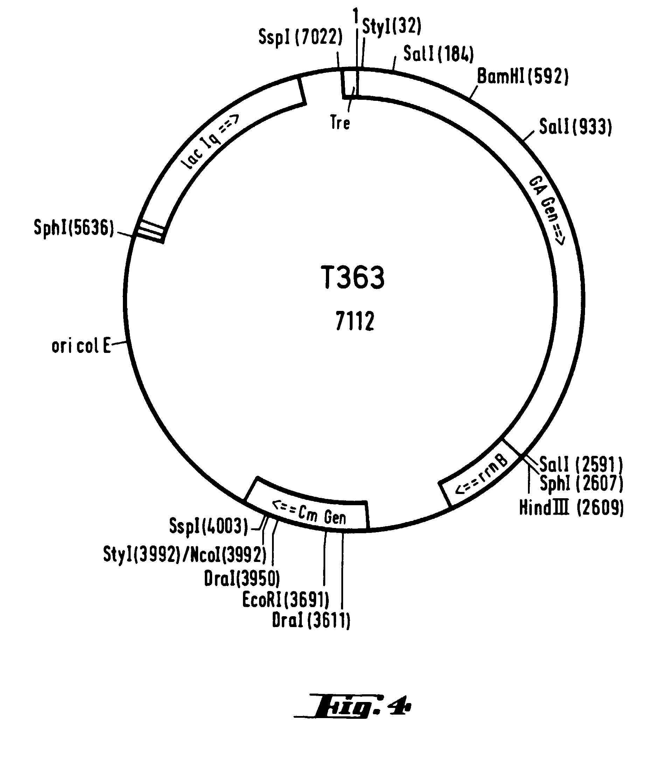

Optimale Ausbeuten wurden mit rekombinanten E. coli Klonen erzielt, die das Plasmid T 363 tragen, dessen Restriktionskarte in Figur 4 gezeigt ist.

### Beispiel 11: Fermentation der E. coli Stäme MC 1061 (T 347) und MC 1061 (T 363)

### Anzuchtsbedingungen:

Alle Anzuchten wurden, mit Ausnahme der zu variierenden Parameter, unter folgenden und für beide Klone identischen Bedingungen durchgeführt:
Die Stammhaltung der Klone erfolgt bei -18 ° C im YT-Glycerin-Medium:

| | |
|---|---|
| Glycerin | 17,0 % |
| Hefeextrakt | 0,7 % |
| Bactotrypton | 0,4 % |
| NaCl | 0,4 % |
| Chloramphenicol | 25 µg/ml |

Aus dieser Suspension werden Agarplatten des gleichen Mediums angelegt, 24 Stunden bei 28 ° inkubiert und die Vorkultur (VK) mit einer Einzelkolonie inokuliert.

### VK-Medium: (NL 5295)

| | |
|---|---|
| Bactopepton | 0,1 % |
| Hefeextrakt | 0,5 % |
| NaCl | 0,5 % |
| Chloramphenicol | 25 µg/ml |
| pH = 7,2 | |

100 ml dieser Nährlösung in 300 ml Erlenmeyerkolben werden nach dem Animpfen für 24 Stunden bei 28 ° C und 220 Upm inkubiert. Die Kultur zeigt dann eine OD₅₇₈ₙₘ von 6,0 - 8,0.

Aus dieser VK wird die folgende Hauptkultur (HK) mit 5 - 10 % (bezogen auf VK mit OD₅₇₈ₙₘ = 3,0) beimpft:
- HK:: NL 5292 + 1 ml Desmophen
20,0 g/l Hefeextrakt (Oxoid)
1,2 g/l NaH₂PO₄ × H₂O
8,5 g/l Na₂HPO₄ × 2H₂O
1,0 g/l KCl
2,0 g/l MgSO₄ × 7H₂O (separat autoklaviert)
0,25 g/l Zitronensäure
5,0 g/l NH₄Cl
4,0 ml/l SLA 5029
0,005 g/l Thiamin → sterilfiltriert (5 mg/10 ml → 0,5/50 ml Nl)
pH = 6,5

### Ferm.beding.:

| | |
|---|---|
| Temp.: | 28 ° C |
| Vol.: | 3,5 L |
| vvm: | 0,75 |
| Upm: | 500 (r = 7 cm) |
| pH: | 7,0 ± 0,2 (mit NH₄OH 25 %ig konstanthalten) |

### Fedbatch:

| | |
|---|---|
| Glycerinlösung: | 525 g Glycerin (99 %)/L HK-Medium (ohne Hefeextrakt und NH₄Cl) |
| Fütterungsrate: | 3,4 ml/L*Std. bei kontinuierlicher Zugabe (max. Glycerinkonz. im Fermenter: 0,2 %) bzw. Einmalzugabe |
| Fütterungsbeginn: | bei pO₂ = 40 - 50 % bzw. OD₅₇₈ₙₘ = 7 - 9 nach ca. 7 Std. Fermentationsdauer |
| Fütterungsdauer: | 40 - 60 Stunden |
| pO₂: | bei ca. 40 % konstanthalten |

- Induktion:: nach Erreichen einer Biomasse von 70 - 80 g feucht/L nach ca. 20 Stunden erfolgt Induktion mit IPTG (1mM Endkonz.). Danach sollte die Glycerinfütterung noch 4 - 40 Stunden fortgesetzt werden.
- Ernte:: 24 - 48 Stunden nach Induktion.

### GA-Expression in E. coli T 347

Mit dem Klon T 347 wurde eine Fedbatch-Fermentation entwickelt, die als Zielsetzung neben einer Erhöhung der Biomasse, den Nachweis der Induzierbarkeit der GA hatte.
Dazu wurden verschiedene Zufütterungsstrategien für Glycerin und deren Auswirkungen auf die IPTG-Induktion untersucht:

| Zugabemodus für Glycerin | Feuchtbiomasse | GA-Aktivität | |
|---|---|---|---|
| | g/L | U/L | U/g |
| keine Zufütterung | 14 | 490 | 35 |
| 1 % Glycerin | 33 | 1219 | 37 |
| 1 % Glycerin + 1 mM IPTG | 41 | 2400 | 59 |
| kontinuierl. Glycerin (3,5 %) + 1 mM IPTG | 61 | 3800* | 62 |

| | | | |
|---|---|---|---|
| * GA zu 40 % intrazellulär | | | |

Obige Tabelle verdeutlicht die gute Korrelation von Biomasse- und GA-Zunahme und zeigt die Verstärkung der GA-Expression durch IPTG, welche nicht durch Ammonium, jedoch durch Glycerin und Glucose in Konzentrationen größer 0,2 % reprimiert wird. Als ausreichende Induktorkonzentration erwies sich 1 mM IPTG. Der optimale Induktionszeitpunkt liegt beim Übergang der Kultur zur stationären Wachstumsphase.

Untersuchungen zur Plasmidstabilität dieses Konstruktes ergaben, daß zum Zeitpunkt der Induktion (ca. 40 Fermentationsstunden) nur noch 60 % der Klone das Plasmid enthielten und, daß nach Induktorzugabe das Plasmid innerhalb von 24 Stunden verloren geht. In der kontinuierlichen Kultur wurde eine Plasmidhalbwertszeit von <50 Stunden ermittelt.

### GA-Expression in E. coli T 363

Der Klon T 363 besitzt ein Plasmid, welches nach 72-stündiger Fermentation noch in allen Zellen vorhanden ist. Dies hat zur Folge, daß deutlich höhere GA-Titer in der Fermentation erreicht werden. Unter oben angeführten optimalen Fedbatchbedingungen können bis zu 100 g Feuchtmasse mit 7000 - 12000 U GA/L KL in ca. 70 Stunden produziert werden. Maximal 40 % dieser Aktivität sind cytoplasmatisch lokalisiert.

Um abzuklären, welche Faktoren die Induktion beeinflussen, wurden folgende Parameter eingehender untersucht:
1 ) Fermentationstemperatur
   Im Bereich von 25 - 28 ° C werden bei maximaler spezifischer Aktivität die höchsten Volumenausbeuten erzielt. Bei 37 ° C wächst der Klon nur noch zu deutlich geringeren Biomassen und exprimiert nur noch maximal 20 U GA/L. Ein Temperaturshift von 37 ° auf 28 ° C zum Zeitpunkt der Induktion führt ebenfalls nicht zur Expression der GA.
2) Komplexe Stickstoffquellen
   Es konnte gezeigt werden, daß bezüglich GA-Volumenproduktivität der Hefeextrakt von Oxoid durch den von Bio Springer oder Marcor ersetzt werden kann. Von den anderen komplexen N-Quellen liefern die NZ-Amine A, B, E und L der Fa. Sheffield sowie Lactalbumin, Soypepton und Bactopepton noch 50 - 70 % der Volumenaktivität. Bezüglich spezifischer GA-Aktivität werden mit den NZ-Aminen, Lactalbumin und Bactopepton in der Regel deutlich höhere Werte erreicht. Hefeextrakt (15 - 30 g/L) ist die z. Z. beste komplexe N-Quelle.
3) Induktorkonzentration
   Untersuchungen im Bereich von 1 - 10 mM IPTG ergaben, daß 1 mM Induktor für die beste GA-Expression ausreichend, höhere Konzentrationen jedoch nicht nachteilig sind.
4) Sauerstoffversorgung
   Zum Zeitpunkt der Induktion hat E. coli T 363 ca. 80 % seiner Biomasse gebildet und der Sauerstoffbedarf liegt bei ca. 50 mMol/L*Std. Dazu wird der pO₂ bei 40 % Sättigung gehalten. Für die Induktion selbst spielt der pO₂ nur eine geringe Rolle, solange er nur über 10 % der Sättigung bleibt. Beispiel 12: Fermentation von E. coli K12 (GA)

- Stamm:: E. coli K12 W3110M: Delta M 15 lac I^{q} pT 363/33
- Stammhaltung:: Ampullenlagerung (16,67 % Glycerinstocks in Medium LB 2fach) bei -74 °C (Tiefkühltruhe)

### Medium LB 2fach:

| | |
|---|---|
| Bacto-Trypton (Difco) | 20 g/l |
| Bacto-Hefe-Extrakt (Difco) | 10 g/l |
| NaCl | 5 g/l |

- Schüttelkultur:: 1000 ml Medium LB 2fach (in 2 1 Stahlkolben) mit 25 mg Chloramphenicol (über Sterilfilter nach Sterilisation des Mediums zugegeben) werden mit dem Inhalt einer Ampulle beimpft und bei 28 ° C auf dem Schüttler (250 Upm, Amplitude 1,25 cm) 4 - 5 h bis zur OD 1,0 bebrütet.
- Vorstufe:: Der DT-Fermenter (200 1 Arbeitsvolumen) wird mit einer 1 Schüttelkultur (OD 1,0) 0,5 %ig beimpft. Nach ca. 6 h Wachstum ist eine OD von 3 - 4 zur Verimpfung auf die Hauptstufe erreicht.

| Vorstufenmedium: | |
|---|---|
| Hefe Extrakt (Bio Springer) | 20,00 g/l |
| Na₂HPO₄ | 0,24 g/l |
| NaH₂PO₄ | 1,70 g/l |
| KCl | 0,20 g/l |
| MgSO₄ * 7H₂O | 0,40 g/l |
| Zitronensäure | 0,05 g/l |
| (NH₄)₂ SO₄ | 1,00 g/l |
| Thiamin/HCl | 1,00 mg/l |
| Spurenelement-Lsg. 5029 | 0,03 ml/l |
| Desmophen 3600 | 0,18 ml/l |

| Spurenelement-Lösung 5029: | |
|---|---|
| CoCl₂ * 6 H₂O | 2,00 g/l |
| NiCl₂ * 2 H₂O | 0,08 g/l |
| CuCl₂ * 2 H₂O | 0,08 g/l |
| ZnCl₂ | 0,80 g/l |
| H₃BO₃ | 4,00 g/l |
| Na₂MoO₄ * 2 H₂O | 2,40 g/l |
| FeSO₄ * 7 H₂O | 1,60 g/l |
| pH-Wert mit HCI auf 2,5 einstellen EDTA | 0,40 g/l |

Die folgenden Bedingungen werden während der Vorstufenfermentation eingehalten: Temperatur: 37 ° C; Druck: 1,0 bar; Zuluft: 7 Nm3/h; Drehzahl: (max. = 175 Upm); pH: 7,2 mit NH₃-Gas halten (vor dem Beimpfen wird der pH-Wert des Mediums von ca. 3,0 - 3,5 auf 7,2 hochgestellt).
- Hauptstufe:: Der ET-Fermenter (2 m³ Arbeitsvolumen) wird 10 %ig mit DT-Kulturbrühe angeimpft. Die Fermentationsdauer beträgt 60 - 80 Stunden.

| Hauptstufenmedium: | |
|---|---|
| Hefe Extrakt (Bio Springer) | 20,00 g/l |
| Na₂HPO₄ | 1,20 g/l |
| NaH₂ PO₄ | 8,50 g/l |
| KCl | 1,00 g/l |
| MgSO₄ * H₂O | 2,00 g/l |
| Zitronensäure | 0,25 g/l |
| (NH₄)₂SO₄ | 5,00 g/l |
| Thiamin/HCl | 5,00 mg/l |
| Spurenelement-Lsg. 5029 | 0,50 ml/l |
| Desmophen 3600 | 0,30 ml/l |

Folgende Fermentationsbedingungen werden eingestellt:

| | |
|---|---|
| Temperatur: | 28 ° C |
| Druck: | 1,0 bar |
| Leistungseintrag: | 2,5 kW/m³ |
| Drehzahl: | 120 Upm (bei einem Durchmesser der Turborührer von 600 mm); |
| Zuluft: | 80 Nm³/h (0,67 vvm) |
| pH-Wert: | 7,2 (geregelt über die Glucose-Nachgabe) |
| Induktion: | mit 1 mM IPTG bei OD 50 |
| Nachgabe: | Glucose (30 %ige Lösung); Beginn zur 6 - 8 h; Menge: 0,5 g/l und h bis 2,5 g/l und h (abhängig vom pH-Verlauf). |

In der Anwachsphase nutzt der Stamm zunächst den Hefe-Extrakt und wächst mit Beginn der Glucose-Zudosierung exponentiell bis OD 50 weiter. Dabei sinkt der Sauerstoffpartialdruck auf 30 bis 10 % ab. Um den Stamm während des exponentiellen Wachstums ausreichend mit Sauerstoff zu versorgen (pO₂ ≧ 30 %), kann der Leistungseintrag durch Erhöhung der Drehzahl und/oder Anhebung der Zuluftmenge auf 3,5 kW/m³ oder mehr gesteigert werden.

Der pH-Wert wird in dieser ersten Fermentationsphase durch stufenweise oder kontinuierliche Anhebung der Glucose-Zudosierung bei 7,2 gehalten.

Die zweite Phase der Fermentation beginnt mit Induktion der Produktbildung durch Zugabe von 1 mM IPTG (Isopropylthiogalaktosid) zur Kulturbrühe (OD 50 ca. 20 - 24 h).

Im weiteren Verlauf der Fermentation wird das Produkt (Glutaryl-Amidase) mit einer durchschnittlichen Bildungsrate von 150 - 200 E/l und Stunde synthetisiert. Dabei geht der Stamm in ein lineares Wachstum über. Die Glucose-Nachdosierung wird bei konstant zu haltendem pH (7,2) auf etwa 0,5 g/l und h herabgesetzt.

Nach 60 - 80 h Laufzeit kann die Fermentation bei einer Biomasseentwicklung von 80 - 100 g/l Feuchtgewicht (Trockenmasse 22 - 25 g/l) und einer Produktbildung von 7.000 - 10.000 E/I beendet werden.

SEQ ID NO: 1
- ART DER SEQUENZ:: Nucleotid mit entsprechendem Protein
- SEQUENZLÄNGE:: je 33 Basen
- STRANGFORM:: Doppelstrang mit überstehenden Erkennungssequenzen
- TOPOLOGIE:: linear
- ART DES FRAGMENTS:: N-terminales Fragment (Signalsequenz)
- HERKUNFT::
- synthetische DNA

C ATG CTG AGA GTT CTG CAC CGG GCG GCG TCC GC
GAC TCT CAA GAC GTG GCC CGC CGC AGG CGG AAC

Met Leu Arg Val Leu His Arg Ala Ala Ser Ala Leu
-29 -25 -20 -18

## Patentansprüche (Patentansprüche für folgende(n) Vertragsstaat(en): AT, BE, CH, DE, DK, FR, GB, GR, IT, LI, LU, NL, SE)

1. Gentechnisches Verfahren zur Herstellung von Glutarylacylase (GA) in E. coli, dadurch gekennzeichnet, daß das in dem Plasmid pCM145 (DSM 6409) enthaltene GA-Gen zur Expression gebracht wird.

2. Verfahren nach Anspruch 1, dadurch gekennzeichnet, daß das GA-Gen unter der Kontrolle eines E. coli-Promotors exprimiert wird.

3. Verfahren nach Anspruch 2, dadurch gekennzeichnet, daß der Promotor induzierbar ist.

4. Verfahren nach Anspruch 3, dadurch gekennzeichnet, daß die Induktion in der logarithmischen Wachstumsphase erfolgt.

5. Verfahren nach Anspruch 4, dadurch gekennzeichnet, daß die Induktion in der späten logarithmischen Phase erfolgt.

6. Verfahren nach einem oder mehreren der Ansprüche 1 bis 5, dadurch gekennzeichnet, daß das GA-Den in einem "low copy number vector" vorliegt.

7. Verfahren nach einem oder mehreren der Ansprüche 1 bis 5, dadurch gekennzeichnet, daß das GA-Gen in einem Expressionsvektor vorliegt, der als Selektionsgen kein Gen für ein sekretiertes, periplasmatisch lokalisiertes Enzym enthält.

8. Verfahren nach Anspruch 7, dadurch gekennzeichnet, daß der Expressionsvektor ein "High Copy Number Vector" ist.

9. Verfahren nach einem oder mehreren der Ansprüche 1 - 5, dadurch gekennzeichnet, daß das GA-Gen in einem "High Copy Number Vector" vorliegt, wobei die Expression des Selektionsgens streng reguliert ist.

10. Verfahren nach einem oder mehreren der Ansprüche 1 bis 9, dadurch gekennzeichnet, daß die Expression bei einer Fermentationstemperatur von etwa 25 bis 30 ° C erfolgt.

11. Plasmid pCM145 (DSM 6409).

12. GA-Gen, enthalten im Plasmid pCM145 (DSM 6409).

13. Genkonstruktionen, enthaltend das Gen nach Anspruch 12.

## Patentansprüche (Patentansprüche für folgende(n) Vertragsstaat(en): ES)

1. Gentechnisches Verfahren zur Herstellung von Glutarylacylase (GA) in E. coli, dadurch gekennzeichnet, daß das in dem Plasmid pCM145 (DSM 6409) enthaltene GA-Gen zur Expression gebracht wird.

2. Verfahren nach Anspruch 1, dadurch gekennzeichnet, daß das GA-Gen unter der Kontrolle eines E. coli-Promotors exprimiert wird.

3. Verfahren nach Anspruch 2, dadurch gekennzeichnet, daß der Promotor induzierbar ist.

4. Verfahren nach Anspruch 3, dadurch gekennzeichnet, daß die Induktion in der logarithmischen Wachstumsphase erfolgt.

5. Verfahren nach Anspruch 4, dadurch gekennzeichnet, daß die Induktion in der späten logarithmischen Phase erfolgt.

6. Verfahren nach einem oder mehreren der Ansprüche 1 bis 5, dadurch gekennzeichnet, daß das GA-Gen in einem "low copy number vector" vorliegt.

7. Verfahren nach einem oder mehreren der Ansprüche 1 bis 5, dadurch gekennzeichnet, daß das GA-Gen in einem Expressionsvektor vorliegt, der als Selektionsgen kein Gen für ein sekretiertes, periplasmatisch lokalisiertes Enzym enthält.

8. Verfahren nach Anspruch 7, dadurch gekennzeichnet, daß der Expressionsvektor ein "High Copy Number Vector" ist.

9. Verfahren nach einem oder mehreren der Ansprüche 1 - 5, dadurch gekennzeichnet, daß das GA-Gen in einem "High Copy Number Vector" vorliegt, wobei die Expression des Selektionsgens streng reguliert ist.

10. Verfahren nach einem oder mehreren der Ansprüche 1 bis 9, dadurch gekennzeichnet, daß die Expression bei einer Fermentationstemperatur von etwa 25 bis 30° C erfolgt.

## Claims (Claims for the following Contracting State(s): AT, BE, CH, DE, DK, FR, GB, GR, IT, LI, LU, NL, SE)

1. A genetic engineering process for the preparation of glutarylacylase (GA) in E. coli, which comprises bringing about the expression of the GA gene contained in the plasmid pCM 145 (DSM 6409).

2. The process as claimed in claim 1, wherein the GA gene is expressed under the control of an E. coli promoter.

3. The process as claimed in claim 2, wherein the promoter is inducible.

4. The process as claimed in claim 3, wherein the induction takes place in the logarithmic phase of growth.

5. The process as claimed in claim 4, wherein the induction takes place in the late logarithmic phase.

6. The process as claimed in one or more of claims 1 to 5, wherein the GA gene is present in a low copy number vector.

7. The process as claimed in one or more of claims 1 to 5, wherein the GA gene is present in an expression vector which contains no gene for a secreted enzyme which is located in the periplasm as selection gene.

8. The process as claimed in claim 7, wherein the expression vector is a high copy number vector.

9. The process as claimed in one or more of claims 1-5, wherein the GA gene is present in a high copy number vector with expression of the selection gene being strictly regulated.

10. The process as claimed in one or more of claims 1 to 9, wherein the expression takes place at a fermentation temperature of about 25 to 30°C.

11. The plasmid pCM 145 (DSM 6409).

12. The GA gene contained in the plasmid pCM 145 (DSM 6409).

13. A gene construction containing the gene as claimed in claim 12.

## Claims (Claims for the following Contracting State(s): ES)

1. A genetic engineering process for the preparation of glutarylacylase (GA) in E. coli, which comprises bringing about the expression of the GA gene contained in the plasmid pCM 145 (DSM 6409).

2. The process as claimed in claim 1, wherein the GA gene is expressed under the control of an E. coli promoter.

3. The process as claimed in claim 2, wherein the promoter is inducible.

4. The process as claimed in claim 3, wherein the induction takes place in the logarithmic phase of growth.

5. The process as claimed in claim 4, wherein the induction takes place in the late logarithmic phase.

6. The process as claimed in one or more of claims 1 to 5, wherein the GA gene is present in a low copy number vector.

7. The process as claimed in one or more of claims 1 to 5, wherein the GA gene is present in an expression vector which contains no gene for a secreted enzyme which is located in the periplasm as selection gene.

8. The process as claimed in claim 7, wherein the expression vector is a high copy number vector.

9. The process as claimed in one or more of claims 1-5, wherein the GA gene is present in a high copy number vector with expression of the selection gene being strictly regulated.

10. The process as claimed in one or more of claims 1 to 9, wherein the expression takes place at a fermentation temperature of about 25 to 30°C.

## Revendications (Revendications pour l'(les) Etat(s) contractant(s) suivant(s): AT, BE, CH, DE, DK, FR, GB, GR, IT, LI, LU, NL, SE)

1. Procédé de génie génétique pour la production de la glutarylacylase (GA) dans *E. coli,* caractérisé en ce que l'on amène à l'expression le gène de GA contenu dans le plasmide pCM145 (DSM 6409).

2. Procédé selon la revendication 1, caractérisé en ce que le gène de GA est exprimé sous le contrôle d'un promoteur de *E. coli.*

3. Procédé selon la revendication 2, caractérisé en ce que le promoteur est inductible.

4. Procédé selon la revendication 3, caractérisé en ce que l'induction s'effectue dans la phase logarithmique de croissance.

5. Procédé selon la revendication 4, caractérisé en ce que l'induction s'effectue dans la phase logarithmique tardive.

6. Procédé selon une ou plusieurs des revendications 1 à 5, caractérisé en ce que le gène de GA est présent dans un vecteur "à faible nombre de copies".

7. Procédé selon une ou plusieurs des revendications 1 à 5, caractérisé en ce que le gène de GA est présent dans un vecteur d'expression qui ne contient comme gène de sélection aucun gène codant pour une enzyme sécrétée, à localisation périplasmique.

8. Procédé selon la revendication 7, caractérisé en ce que le vecteur d'expression est un vecteur "à grand nombre de copies".

9. Procédé selon une ou plusieurs des revendications 1 à 5, caractérisé en ce que le gène de GA est présent dans un vecteur "à grand nombre de copies", l'expression du gène de sélection étant strictement régulée.

10. Procédé selon une ou plusieurs des revendications 1 à 9, caractérisé en ce que l'expression s'effectue à une température de fermentation d'environ 25 à 30°C.

11. Plasmide pCM145 (DSM 6409).

12. Gène de GA contenu dans le plasmide pCM145 (DSM 6409).

13. Produits de construction génique, contenant le gène selon la revendication 12.

## Revendications (Revendications pour l'(les) Etat(s) contractant(s) suivant(s): ES)

1. Procédé de génie génétique pour la production de la glutarylacylase (GA) dans *E. coli,* caractérisé en ce que l'on amène à l'expression le gène de GA contenu dans le plasmide pCM145 (DSM 6409).

2. Procédé selon la revendication 1, caractérisé en ce que le gène de GA est exprimé sous le contrôle d'un promoteur de *E*. *coli.*

3. Procédé selon la revendication 2, caractérisé en ce que le promoteur est inductible.

4. Procédé selon la revendication 3, caractérisé en ce que l'induction s'effectue dans la phase logarithmique de croissance.

5. Procédé selon la revendication 4, caractérisé en ce que l'induction s'effectue dans la phase logarithmique tardive.

6. Procédé selon une ou plusieurs des revendications 1 à 5, caractérisé en ce que le gène de GA est présent dans un vecteur "à faible nombre de copies".

7. Procédé selon une ou plusieurs des revendications 1 à 5, caractérisé en ce que le gène de GA est présent dans un vecteur d'expression qui ne contient comme gène de sélection aucun gène codant pour une enzyme sécrétée, à localisation périplasmique.

8. Procédé selon la revendication 7, caractérisé en ce que le vecteur d'expression est un vecteur "à grand nombre de copies".

9. Procédé selon une ou plusieurs des revendications 1 à 5, caractérisé en ce que le gène de GA est présent dans un vecteur "à grand nombre de copies", l'expression du gène de sélection étant strictement régulée.

10. Procédé selon une ou plusieurs des revendications 1 à 9, caractérisé en ce que l'expression s'effectue à une température de fermentation d'environ 25 à 30°C.
